# EUROPÄISCHE PATENTSCHRIFT

(11) **EP 2 705 828 B1**
(45) Veröffentlichungstag und Bekanntmachung des Hinweises auf die Patenterteilung: **14.02.2018**
(21) Anmeldenummer: 12183046.7
(22) Anmeldetag: 05.09.2012
(51) Int. Cl.: A61K 8/31, A61K 8/34, A61K 8/37, A61K 8/44, A61K 8/49, A61K 8/81, A61Q 19/00, A61K 8/92

(54) **Schnell einziehende, hautpflegende Softcreme**
Quick-penetrating, soft skin care cream
Crème de soin pour la peau à pénétration rapide

(43) Veröffentlichungstag der Anmeldung: 12.03.2014
(73) Patentinhaber: Deb IP Limited, Denby, Derbyshire DE5 8JZ (GB)
(72) Erfinder: Heisler, Eckhard, 47608 Geldern (DE); Hemming, Markus, 46045 Oberhausen (DE); Drewes, Carina, 47798 Krefeld (DE); Wans, Nicole, 47807 Krefeld (DE)
(74) Vertreter: Elsy, David

(56) Entgegenhaltungen:
- EP-A1- 1 325 736
- WO-A1-03/017952
- "Wax", Wikipedia, 5. April 2013 (2013-04-05), Seiten 1-5, XP55062323, Gefunden im Internet: URL:http://en.wikipedia.org/wiki/Wax [gefunden am 2013-05-08]
- "PEG-30 Glyceryl Cocoate" In: "International Cosmetic Ingredient Dictionary and Handbook", 2010, Personal Care Products Council, XP002697161, Bd. 2, Seiten 2058-2059, * Abschnitt "Reported Functions" *

## Beschreibung

Die Erfindung liegt auf dem Gebiet der kosmetischen und/oder dermatologischen Zusammensetzungen und betrifft schnell einziehende, hautpflegende Zusammensetzungen zur topischen Anwendung auf der menschlichen Haut enthaltend Wachskörper, Carbomere, bestimmte Fettalkohole, Emollients, bestimmte Ester aus Fettsäure und Fettalkohol und mindestens einen Wirkstoff ausgewählt aus Creatin, Allantoin und/oder Glycerin. Die Erfindung betrifft weiterhin Verfahren zur Herstellung und Verwendungen solcher Zusammensetzungen.

Die menschliche Haut ist mit einer Oberfläche von ca. 1,5 bis 2,0 m² das oberflächengrößte Organ des Menschen, das für den Körper lebenswichtige Funktionen wahrnimmt. Hierfür enthält die Haut Blut- und Lymphgefäße, durch deren Wände hindurch der Austausch von Lymphflüssigkeit, Gasen, Nähr- und Abfallstoffen stattfinden kann, um z.B. Ernährung und Stoffwechsel zu gewährleisten. Weitere Funktionen der Haut sind die Regelung der Körpertemperatur, der Schutz des Körpers gegen Austrocknung und gegen äußere mechanische, chemische und bakterielle Einwirkungen. So halten die Sekrete von Hauttalgdrüsen die Haut geschmeidig und helfen bei der Regulierung des Wasserhaushaltes der Haut. Darüber hinaus vermittelt die Haut unter anderem über freie Nervenendigungen dem Organismus Tast-, Wärme- und Kälte- und Schmerzreize und hat somit die Funktion eines Sinnesorgans.

Die mannigfaltigen für Hygiene, Körperpflege und Kosmetik angebotenen Produkte, wie z.B. Hautreinigungs-, Hautschutz- und Hautpflegeprodukte dienen daher nicht nur primär der Reinigung, dem Schutz bzw. der Pflege der Haut, sondern sie erhalten auch die Lebensqualität und sichern das Wohlbefinden des Menschen.

Als Körperhülle stellt die menschliche Haut die Verbindung, aber zugleich auch Abgrenzung des menschlichen Körpers mit seiner Außenwelt dar. Insbesondere erfolgt über sie die Kontaktaufnahme mit allem, was der menschliche Organismus zum Leben benötigt - aber auch was sie gefährden kann.

Daher ist es insbesondere wichtig, die menschliche Haut zu pflegen und sie vor Umwelteinflüssen zu schützen. Die Vergangenheit von Hautpflegeprodukten lässt sich daher weit in die menschliche Entwicklungsgeschichte zurück verfolgen. Da die Anforderungen an Hautpflegeprodukte sehr unterschiedlich sein können, wurde in den vergangenen Jahren das Angebot an unterschiedlichen Hautpflegeprodukten immer breiter, um sich auf Unterschiedliche Bedürfnisse der Verbraucher und unterschiedliche Beanspruchung der Haut in Bezug auf das jeweilige Umfeld besser anpassen zu können. Insbesondere an Hautpflegeprodukte zur Nutzung am Arbeitsplatz werden deutlich breitere Anforderungen gestellt, die über den Rahmen von haushaltsgebräuchlichen Hautpflegeprodukten weit hinaus gehen. Gemäß W. Dicke (siehe W. Dicke; I. Funk-Stendel; B. Marschner, F. Zuther: Alles über Hautschutz, Hautreinigung, Hautpflege. 5. aktualisierte und erweiterte Auflage (115-118); Oktober 2005) sind moderne Hautpflegemittel Zubereitungen, die zur Erhaltung und Wiederherstellung der physiologischen Hydrolipidemulsion in den oberen Hautschichten beitragen. Hautpflegemittel enthalten häufig spezielle Inhaltsstoffe, die den Regenerationsprozess der Haut unterstützen. Dabei müssen Produkte zur Pflege und/oder Gesunderhaltung der Haut, wie auch Hautschutzprodukte, zum Beispiel an die Anforderungen des Arbeitsplatzes und an den Grad der Beanspruchung der Haut angepasst sein.

Da der Haut durch wiederholtes Händewaschen oder bei Arbeit in feuchtem Milieu, hauteigene Lipide und natürliche Feuchthaltefaktoren entzogen werden, ist die Zufuhr von Feuchtigkeit bei der Hautpflege von zentraler Bedeutung. In diesem Zusammenhang haben Feuchthaltefaktoren die vornehmliche Aufgabe, Feuchtigkeit in der Haut zu binden und ein Verdunsten des Wassers aus der Haut zu verlangsamen. Werden der Haut diese Komponenten entzogen, kommt es zu einer vermehrten Verdunstung von Wasser und die Haut vermittelt einen trockenen und entfetteten Eindruck, der auch als Vorstufe einer Abnutzungs-Dermatose angesehen werden kann. Daher werden in Hautpflegemitteln neben Lipiden und Feuchtigkeit häufig auch wasserbindende Faktoren wie Milchsäure eingesetzt. Handelt es sich bei dem Hautpflegemittel um eine Öl-in-Wasser-Emulsion, werden spezielle Anforderung an die Formulierung gestellt, weil es bei Anwendung stark wasserhaltiger Emulsionen durch einen sogenannten "Dochteffekt" zusätzlich zu einer Austrocknung der Haut kommen kann. Dem muss bei der Entwicklung eines Hautpflegeproduktes durch die Kombination unterschiedlicher Feuchthaltefaktoren Rechnung getragen werden. Schlussendlich spielt im Hinblick auf die potentielle Anwendung eines Hautschutzmittels die Akzeptanz des Produktes beim Endverbraucher eine übergeordnete Rolle. Hierbei stehen insbesondere Parameter wie das Einziehvermögen, das Abdruckverhalten, Griffsicherheit nach der Anwendung, Fettglanz und auch der Geruch im Vordergrund.

Für die Entscheidung eines Betriebs für ein Hautpflegeprodukt zur Nutzung am Arbeitsplatz spielen darüber hinaus noch ganz andere Parameter eine ausschlaggebende Rolle. Neben den Eigenschaften bezüglich Pflege und Schutz der Haut vor alltäglichen Arbeitseinflüssen, ist beispielsweise das Verhalten des entsprechenden Hautpflegeproduktes nach der Applikation entscheidend. Die behandelten Hautflächen, insbesondere Hände und Unterarme, kommen nach der Anwendung mit dem Hautpflegeprodukt unweigerlich mit dem Arbeitsumfeld in Kontakt. Dies hat zur Folge, dass Oberflächen jeglicher Art am Arbeitsplatz durch den Kontakt mit behandelten Hautflächen mit unerwünschten Rückständen aus den Hautpflegeprodukten kontaminiert werden können. Dies ist insbesondere dann kritisch, wenn der Kontakt mit empfindlichen Oberflächen, Produkten oder Produktionsanlagen erfolgt. Im schlechtesten Fall wirken sich Effekte, die beispielsweise hydrophile oder emulgierende Bestandteile von Hautpflegeprodukten auf wasserabweisende Oberflächen haben, derart auf die betreffende Oberfläche aus, dass Veränderungen, z.B. in Farbgebung, Konsistenz oder auch chemischer Zusammensetzung von Oberflächen hervorgerufen werden können. Darüber hinaus können Rückstände auf Werkstücken oder Zwischenprodukten negative Auswirkung auf das Folgeprodukt haben. Außerdem kann das Eincremen der Hände mit Hautpflegeprodukten die Griffigkeit beeinflussen und dadurch das Arbeiten mit Werkzeugen gefährlicher machen.

WO 03/017952 A1 offenbart kosmetische Öl-in-Wasser Zubereitungen und ihre Verwendung zur leichten Hautpflege. Es besteht daher weiterhin Bedarf an Hautpflegeprodukten, die Einerseits den hohen Anforderungen des Verbrauchers an Pflege und Schutz genüge leisten und andererseits die Anforderungen der Betriebe an eine universale Einsetzbarkeit, ohne übermäßige Rücksichtnahme auf eventuell kritisches Arbeitsumfeld und empfindliche Oberflächen, entsprechen.
Die erfindung ist wie in den Ansprüchen beschrieben. Aufgabe der vorliegenden Erfindung war es daher, Zusammensetzungen zur topischen Anwendung auf der Haut bereitzustellen, die eine universale Einsetzbarkeit im Arbeitsumfeld gewährleisten, und damit eine höhere Akzeptanz bei der täglichen Benutzung in Betrieben erreichen; die hervorragende Pflegeeigenschaften aufweisen und gleichzeitig geeignete Eigenschaften in Bezug auf die Stabilität der Zusammensetzung besitzen.

Es hat sich gezeigt, dass Wirkstoffkombinationen in den genannten Gewichtsbereichen die oben genannte Aufgabe besonders gut lösen. Insbesondere bemerkenswert ist, dass erfindungsgemäße Zusammensetzungen auch bei erhöhter Elektrolytkonzentration, beispielsweise in Anwesenheit von Salzen, wie unter anderen Natrium- und/oder Kaliumsalzen, stabil sind. Erfindungsgemäße Zusammensetzungen weisen ein bemerkenswert gutes Einziehverhalten in die Haut auf und sind somit insbesondere in Betrieben, also bei der täglichen Anwendung zur Pflege und zum Schutz der Haut während der Arbeit, geeignet. Das schnelle Einziehverhalten hat neben dem angenehmen, nicht klebrigen Empfinden durch den Anwender/Verbraucher, den positiven Effekt, dass die Berührung von Oberflächen mit den mit erfindungsgemäßen Zusammensetzungen behandelten Hautpartien zu möglichst wenig Rückständen auf den entsprechenden Oberflächen führt. Dies gilt für den Kontakt von Oberflächen jeglicher Art, auf denen der vorangehende Gebrauch von Hautpflegeprodukten unerwünschte Rückstände hinterlassen kann. Insbesondere wenig Rückstände hinterlassen erfindungsgemäße Zusammensetzungen auf wasserabweisenden Oberflächen wie beispielsweise Kunststoffoberflächen, wie unter anderem auch Tastatur und Telefon, elektronische Bauteile, wie beispielsweise Leiterplatten, Glasoberflächen, Plexiglas oder modifizierte Glasoberflächen, zu lackierende Oberflächen, Lacke, insbesondere Autolacke, Lasuren, Keramik, Porzellan, Metall, Legierungen, Stein, Holz und bearbeitete Holzflächen, Labormaterialien oder mit Polymeren behandelten Oberflächen. Insbesondere beim Einsatz in Betrieben/dem beruflichen Umfeld sind möglichst wenige Rückstände auf Oberflächen wünschenswert, um etwaige Kontamination des jeweiligen Arbeitsumfeldes, wie beispielsweise auch von Werkstücken, Produkten und Produktionsanlagen, zu vermeiden. Durch ein schnelles Einziehverhalten können daher auch negative Effekte, die beispielsweise hydrophile oder emulgierende Bestandteile von Zusammensetzungen auf wasserabweisende Oberflächen, wie beispielsweise zu lackierende Oberflächen, haben, vermieden werden. Somit kann bevorzugt eine ungewünschte Veränderung von Oberflächen, die mit Hautpartien in Berührung kommen, welche mit erfindungsgemäßen Zusammensetzungen behandelten wurden, gegenüber im Stand der Technik bekannten Zusammensetzungen verringert oder vollständig vermieden werden. Zudem können Rückstände auf Werkstücken oder Produkten vermieden oder zumindest vermindert werden, die bei der Weiterverarbeitung negative Auswirkung auf das Folgeprodukt haben könnten. Ebenso wird die Griffigkeit der mit den erfindungsgemäßen Zusammensetzungen behandelten Hände gar nicht oder zumindest nur minimal beeinträchtigt.

Die erfindungsgemäßen Gegenstände werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. pH-Werte wurden, soweit nicht anders angegeben, mit handelsüblichen pH-Metern auf Basis von Potentiometrie bestimmt. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Besonders bevorzugt enthalten die erfindungsgemäßen Zusammensetzungen als Wachskörper a) hydriertes Ricinusöl, das beispielsweise unter der Handelsmarke CUTINA® HR von der Firma Cognis vertrieben wird, bzw. Mineralwachse wie z.B. Ceresin, das als mikrokristallines Wachs unter der Handelsbezeichnung Paracera® W 80 erhalten werden kann. Es hat sich herausgestellt, dass Zusammensetzungen, die als Wachskörper der Komponente a) 0,2 bis 0,8 Gew.-%, vorzugsweise 0,3 bis 0,5 Gew.-% mindestens eines tierischen und/oder pflanzlichen Wachskörpers und mindestens eines chemisch modifizierten Wachskörpers enthalten, ein besonders gutes Einziehverhalten und eine sehr gute Konsistenz aufweisen. Ganz besonders hervorragende Ergebnisse konnten insbesondere dann erzielt werden wenn das Gewichtsverhältnis des Gesamtgewichts der eingesetzten tierischen und pflanzlichen Wachskörper zu dem Gesamtgewicht der eingesetzten chemisch modifizierten Wachskörper zwischen 1:2 und 2:1 liegt und besonders bevorzugt bei 1:1 liegt.

Der Begriff Polymer im Sinne dieser Erfindung umfasst sowohl Homopolymere als auch Copolymere. Bevorzugte Polymere der Komponente b) sind besonders elektrolytresistent und erzielen auch in Zusammensetzungen mit erhöhter Salzkonzentration stabile Zusammensetzungen. Bevorzugte Acrylsäure Polymere im Sinne dieser Erfindung sind alle homopolymere der Acrylsäuren oder der Derivate der Acrylsäure, so wie Copolymere der Acrylsäure und/oder der Derivate der Acrylsäure. Der Begriff Derivat der Acrylsäure umfasst alle chemisch modifizierten Acrylsäuren, insbesondere Ester der Acrylsäure. Besonders bevorzugte Derivate der Acrylsäure sind Alkylester der Acrylsäure, insbesondere bevorzugt C10 bis C30 Alkylester der Acrylsäure. Bevorzugte Polymere der Acrylsäure sind Copolymere aus der Acrylsäure und einem Ester der Acrylsäure. Bevorzugte Homopolymere der Acrylsäure sind Polymere, die beispielsweise unter der INCI Bezeichnung Carbomer bekannt sind. In einer bevorzugten Ausführungsform handelt es sich bei dem Polymer der Komponente b) um Acrylsäure Polymere, wie sie beispielsweise von der Firma Evonik Industries AG unter den Handelsnamen Tego<®> Carbomer 141 oder Tego<®> Carbomer 341 ER vertrieben werden. Diese genannten Ausführungsformen erzielen in Kombination mit den weiteren Wirkstoffen der Wirkstoffgruppen a), c), d), e) und f) eine außergewöhnliche Stabilität der Gesamtzusammensetzung und besonders ausgezeichnetes Einziehverhalten auf der Haut. Besonders bevorzugt handelt es sich um Acrylates/C10-30

Alkyl Acrylate Crosspolymer, das z.B. von der Firma Evonik Industries AG unter dem Handelsnamen Tego<®> Carbomer 341 ER vertrieben wird, da mit diesem Wirkstoff besonders hervorragende Ergebnisse und eine sehr gute Stabilität der Zusammensetzung, bei erstaunlich niedriger Konzentration des Polymers b), beispielsweise bei einer Konzentration von 0,3 bis 0,4 Gew.-%, erzielt werden können. Insbesondere bemerkenswert ist, dass solche Polymere b) auch bei erhöhter Elektrolytkonzentration, beispielsweise in Anwesenheit von Salzen, wie unter anderen Natrium- und/oder Kaliumsalze, stabil sind. In einer besonderen Ausführungsform handelt es sich bei dem Polymer der Komponente b) nicht um homopolymere Acrylsäuren, wie sie beispielsweise von der Firma Evonik Industries AG unter dem Handelsnamen Tego<®> Carbomer 134 oder Tego<®> Carbomer 140 vertrieben werden.

Zusammensetzungen, bei welchen das Gewichtsverhältnis des Gesamtgewichts an Wachskomponente a) zu dem Gesamtgewicht an Polymer der Komponente b) 2:1 bis 1:2, bevorzugt 1:1 beträgt sind erfindungsgemäß eine bevorzugte Ausführungsform, da solche Zusammensetzungen ein ganz besonders hervorragendes Einziehverhalten auf der Haut aufweisen bei gleichzeitig sehr guter Stabilität.

Die erfindungsgemäßen Zusammensetzungen enthalten als Komponente c) 0,1 bis 0,75 Gew.-%, vorzugsweise 0,2 bis 0,3 Gew.%, mindestens eines linearen, gesättigten unsubstituierten Fettalkohols mit 12 bis 20 Kohlenstoffatomen. Erfindungsgemäße Fettalkohole umfassen damit primäre Alkohole mit 12, 13, 14, 15, 16, 17, 18, 19 oder 20 Kohlenstoffatomen, die mit Ausnahme der -CH2-OH Gruppe ausschließlich wasserstoffsubstituiert sind. Bevorzugt sind die Fettalkohole mit 16 und 18 Kohlenstoffatomen, Cetylalkohol und Stearylakohol. Insbesondere bevorzugt ist Stearylakohol. Diese Ausführungsformen erzielen in Kombination mit den weiteren Wirkstoffen der Wirkstoffgruppen a), b), d), e) und f) eine außergewöhnliche Stabilität der Gesamtzusammensetzung und besonders ausgezeichnetes Einziehverhalten auf der Haut und sorgt dafür, dass die Zusammensetzung als nicht wässrig und reichhaltig wahrgenommen wird.

Unter Emollient im Sinne der vorliegenden Erfindung können prinzipiell alle, dem Fachmann bekannten, Wirkstoffe verstanden werden, die unter keine der Definitionen der anderen Komponenten a), b), c), e) und f) fallen und die die Sensorik einer Zusammensetzung positiv beeinflussen, also beispielsweise die Epidermis der Haut geschmeidiger und weicher machen. Die Emollientien sind ausgewählt aus der Gruppe PEG-30 Glyceryl Cocoat, PEG-6 Capryl/Capric Glycerid, Sorbitan Sesquicaprylat, Sucrose Cocoat, Isoamyl Cocoat Polyglyceryl-3 Caprat, PPG-3 Myristyl Ether, PPG-11 Stearyl Ether, Cetearyl Isononanoate, Cetyl Ethylhexanoate, Caprylic / Capric Triglyceride, Decal Cocoate, Diethylhexyl Carbonate, Decyl Oleate, PPG-15 Stearyl Ether, Octyldodecanol, Isocetyl Palmitat, Isohexadecane, Cetearyl Ethylhexanoat, Isopropyl Myristat, Oleyl Erucat, Ethylhexyl Palmitat, Ethylhexyl Stearat, Isopropyl Palmitat, PPG-14 Butyl Ether, Triisostearin, C12-15 Alkylbenzoat, Cetyl Ricenoleate, Glyceryl Ricenoleat, Glyceryl Stearat, Isocetyl Palmitat, Isocetyl stearat, Tocopheryl Linoleat, Methylheptyl Isostearat und Mischungen daraus, insbesondere bevorzugt ist Isoamyl Cocoat. Diese Ausführungsformen erzielen in Kombination mit den weiteren Wirkstoffen der Wirkstoffgruppen a), b), c), e) und f) ein außergewöhnlich gutes Spreitungsverhalten der Gesamtzusammensetzung und besonders ausgezeichnetes Einziehverhalten auf der Haut.

Die erfindungsgemäßen Zusammensetzungen enthalten als Komponente e) 0,5 bis 1,5 Gew.-%, vorzugsweise 0,8 bis 1,2 Gew.-%, mindestens eines Esters mit 20 bis 34 Kohlenstoffatomen aus einer linearen, gesättigten unsubstituierten Fettsäure und einem linearen, gesättigten unsubstituierten Fettalkohol. Erfindungsgemäße Ester aus einer linearen, gesättigten unsubstituierten Fettsäure und einem linearen, gesättigten unsubstituierten Fettalkohol umfassen damit Ester mit 20, 21, 22, 23, 24, 25, 26, 27, 28, 29, 30, 31, 32, 33 oder 34 Kohlenstoffatomen, die mit Ausnahme der -COOCH₂- Gruppe ausschließlich wasserstoffsubstituiert sind. Die Verteilung der Kohlenstoffatome auf die Fettsäurekomponente und die Fettalkoholkomponente kann beliebig erfolgen. Erfindungsgemäß bevorzugt sind solche Ester, bei welchen sich die Anzahl der Kohlenstoffatome in der Fettsäurekomponente und die Anzahl der Kohlenstoffatome in der Fettalkoholkomponente um nicht mehr als 5, bevorzugt nicht mehr als 4 , weiterhin bevorzugt nicht mehr als 3, insbesondere nicht mehr als 2, besonders bevorzugt nicht mehr als 1 unterscheidet. Ganz besonders bevorzugt sind solche Ester, bei welchen die Anzahl der Kohlenstoffatome in der Fettsäurekomponente und die Anzahl der Kohlenstoffatome in der Fettalkoholkomponente gleich ist. Diese Ausführungsformen erzielen in Kombination mit den weiteren Wirkstoffen der Wirkstoffgruppen a), b), c), d) und f) eine außergewöhnliche Stabilität der Gesamtzusammensetzung ein besonders ausgezeichnetes Einziehverhalten und einen besonders seidigen Eindruck auf der Haut. Bevorzugte Ester der Komponente e) können ausgewählt sein aus Cetyl Palmitat, Cetyl Stearat, Stearyl Heptanoate, Myristyl Myristat oder Mischungen daraus. Insbesondere bevorzugt sind Ester der Komponente e) ausgewählt aus Cetyl Palmitat, Cetyl Stearat, Myristyl Myristat oder Mischungen daraus, da sich bei diesen Estern der Komponente e) jeweils die Anzahl der Kohlenstoffatome in der Fettsäurekomponente und die Anzahl der Kohlenstoffatome in der Fettalkoholkomponente um nicht mehr als 2 unterscheidet. Besonders bevorzugt ist weiterhin der Ester Myristyl Myristat mit je 14 Kohlenstoffatomen in der Fettsäurekomponente und in der Fettalkoholkomponente, bei welchem die Anzahl der Kohlenstoffatome in der Fettsäurekomponente und die Anzahl der Kohlenstoffatome in der Fettalkoholkomponente gleich ist.

Das Gesamtgewicht der Wirkstoffe der Komponente f) beträgt bevorzugt 0,2 bis 8 Gew.-%, vorzugsweise 1 bis 6 Gew.%, besonders bevorzugt 3 bis 5 Gew.%, bezogen auf das Gesamtgewicht der Zusammensetzung. Bevorzugt kann Glycerin in Mengen von kleiner 4 Gew.-%, insbesondere 1 bis 4 Gew.-%, eingesetzt werden. Bevorzugt werden Glycerin, Creatin und Allantoin eingesetzt. Diese Ausführungsformen erzielen in Kombination mit den weiteren Wirkstoffen der Wirkstoffgruppen a), b), c), d) und e) eine außergewöhnliche Stabilität der Gesamtzusammensetzung und besonders ausgezeichnetes Einziehverhalten auf der Haut. Zusammensetzungen, bei denen das Gewichtsverhältnis des Gesamtgewichts von Creatin und Allantoin zu dem Gesamtgewicht an Glycerin 1:6 bis 1:2, bevorzugt 1:3,2 bis 1:3,6 beträgt, erzielen einen besonders guten feuchtigkeitsspendenen Effekt auf der Haut. Insbesondere Zusammensetzungen, die als Komponente f) Creatin, Allantoin und Glycerin in den angegebenen Gewichtsverhältnissen enthalten erzielen bemerkenswert gute feuchtigkeitsspendende Effekte.

Für den Fall, dass es Überschneidungen einzelner Wirkstoffe der Wirkstoffgruppen a) bis f) geben sollte, so sind alle Wirkstoffe, bei denen es sich um einen linearen, gesättigten unsubstituierten Fettalkohol mit 12 bis 20 Kohlenstoffatomen handelt der Gruppe c) zuzuordnen und alle Wirkstoffe bei denen es sich um einen Ester mit 20 bis 34 Kohlenstoffatomen aus einer linearen, gesättigten unsubstituierten Fettsäure und einem linearen, gesättigten unsubstituierten Fettalkohol handelt der Gruppe e) zuzuordnen. Eine Zuordnung dieser Wirkstoffe zu anderen Wirkstoffgruppen ist nicht im Sinne dieser Erfindung. Die Zuordnung von Wirkstoffen zu den Wirkstoffgruppen a) bis f) erfolgt erfindungsgemäß in der Reihenfolge f), c), e), b), a), d). Die nachgestellte Wirkstoffgruppe ist nur zu beachten, wenn ein Wirkstoff keiner der vorangehenden Wirkstoffgruppen zugeordnet werden kann. Ansonsten wird der Wirkstoff in die erste, passende und der Reihenfolge entsprechenden Wirkstoffgruppe zugeordnet.

Bevorzugt haben erfindungsgemäße Zusammensetzungen einen pH Wert kleiner 7. Besonders bevorzugt haben die Zusammensetzungen einen pH Wert zwischen 4,5 und 6,0 insbesondere bevorzugt zwischen 5,0 und 5,5.

Bevorzugt weisen die erfindungsgemäßen Zusammensetzungen eine Viskosität von 5000 bis 100000 mPas s, besonders bevorzugt von 8000 bis 50000 mPas s, insbesondere bevorzugt von 10.000 bis 25.000 mPas s gemessen mit einem Viskosimeter der Fa. Brookfield Engineering Inc., nämlich einem Brookfield RVT Rotationsviskosimeter mit Spindelset 1-7.

Diese Zusammensetzungen weisen eine außergewöhnliche Stabilität der Gesamtzusammensetzung und besonders ausgezeichnetes Einziehverhalten auf der Haut auf. Daher hinterlassen sie besonders wenige Rückstände auf

Oberflächen, insbesondere wasserabweisenden Oberflächen. Zudem sind diese erfindungsgemäßen Zusammensetzungen besonders gut verteilbar, nicht klebrig und die Zusammensetzung ist stabil während des Auftragens auf die Haut, d.h. die Zusammensetzung bricht nicht.

Die Zusammensetzung kann neben den Wirkstoffkombinationen der Wirkstoffe a) bis f) gewünschtenfalls weitere Wirkstoffe enthalten.

In einer bevorzugten Ausführungsform enthält die Zusammensetzung weiterhin Urea, bevorzugt in Mengen von 0,0001 bis 5 Gew.-%, insbesondere bevorzugt 0,001 bis 2,5 Gew.-%, bezogen auf das Gesamtgewicht der Zusammensetzung.

Bevorzugt können zudem solche Zusammensetzungen sein, die weiterhin den Wirkstoff Polyglyceryl-3-dicitrate/stearate enthalten.

Bevorzugt sind solche Zusammensetzungen die weiterhin die Wirkstoffe Polyglyceryl-3-dicitrate/stearate und Urea enthalten. In einer besonderen Ausführungsform enthalten erfindungsgemäße Zusammensetzungen Polyglyceryl-3-dicitrate/stearate und Urea in einem Gewichtsverhältnis von 100:1 bis 150:1, bevorzugt von 125:1. Zusammensetzungen, die Polyglyceryl-3-dicitrate/stearate und Urea in diesen Gewichtsverhältnissen enthalten, sind überraschenderweise besonders stabil und wirken besonders feuchtigkeitsspendend auf der Haut bei gleichzeitig sehr gutem Einziehverhalten auf der Haut.

Bevorzugt können zudem solche Zusammensetzungen sein, die weiterhin den Wirkstoff Betain, auch bekannt unter der Bezeichnung Trimethylglycin, enthalten. Bei unverändert guter Stabilität können solche Zusammensetzungen einen besonders intensiven Pflegeeindruck hervorrufen.

Weiterhin bevorzugt sind solche Zusammensetzungen die außerdem die Wirkstoffe Betain, und Urea enthalten. Insbesondere bevorzugt werden Betain und Urea in einem Gewichtsverhältnis von 4:1 bis 1:1, weiter bevorzugt von 2:1 eingesetzt. Zusammensetzungen, die Betain und Urea in diesen Gewichtsverhältnissen enthalten, wirken besonders feuchtigkeitsspendend auf der Haut.

Bevorzugte erfindungsgemäße Zusammensetzungen enthalten mindestens 70 Gew.-%, bevorzugt mindestens 75 Gew.-%, besonders bevorzugt mindestens 80 Gew.-% Wasser. Bei den erfindungsgemäßen Zusammensetzungen handelt es sich vorzugsweise um kosmetische und/oder dermatologische Formulierungen, die im Allgemeinen als wässrige alkoholische Lösungen, Cremes, Emulsion, Lotionen, Gele, Aerosolspray oder -schaum, Nonaerosolspray oder -schaum vorliegen können. Bevorzugt handelt es sich um Cremes und/oder Lotionen. Insbesondere bevorzugt handelt es sich um hydrophile Cremes und/oder Lotionen, die als Öl in Wasser Emulsion (O/W-Emulsion) vorliegen können.

Die erfindungsgemäßen Zusammensetzungen können optional weitere Komponenten enthalten die üblicherweise in kosmetischen und/oder dermatologischen Formulierungen Anwendung finden. Bevorzugte weitere Komponenten können ausgewählt sein aus der Gruppe der Duftstoffe und Parfüme, Konservierungsmittel und/oder pH Stellmittel, wie zum Beispiel wässrige Natronlauge.

Die erfindungsgemäßen Zusammensetzungen können zur Anpassung auch weitere übliche Bestandteile enthalten, welche zur Behandlung, Pflege, Reinigung und dem Schutz der Haut dienen, wie beispielsweise hautkosmetische Wirkstoffe (active ingredients). Von dem Begriff hautkosmetische Wirkstoffe im Sinne dieser Erfindung werden beispeilsweise Ceramide, Pseudoceramide, Proteinhydrolysate pflanzlichen oder tierischen Ursprungs auf Basis Keratin, Collagen, Elastin, Weizen, Reis, Soja, Milch, Seide, Mais, Aminosäuren und Aminosäurederivate, entzündungshemmende Wirkstoffe, antimikriobelle Wirkstoffe, gebräuchliche Antioxidantien, Vitamine, Dexpanthenol, Milchsäure, Pyrrolidoncarbonsäure, Bisabolol sowie Pflanzen-, Hefe- und/oder Algenextrakten umfasst.

Die Kombination mit üblicherweise verwendeten organischen oder anorganischen UV-Filtersubstanzen ist ebenfalls möglich und z. B. in Sonnenschutzpräparaten als besonders vorteilhaft anzusehen, da während der Anwendung das Austrocknen der Haut wirksam unterbunden werden kann und dadurch die natürliche Schutzfunktion der Haut erhalten bleibt. Zusätzlich können noch weitere kosmetische Hilfs- und Zusatzstoffe, die in solchen Zubereitungen üblich sind, enthalten sein. Solche Hilfstoffe sind z. B. Lösungsvermittler wie Ethanol, Isopropanol, Ehtylenglykol; Propylenglykol und Diethylenglykol. Zu den weiteren Komponenten zählen kosmetische Öle pflanzlichen und synthetischen Ursprungs, Silikonöle, Fette, Rückfetter, Emulgatoren, anionische, zwitterionische, ampholytische und nichtionische Tenside und/oder Farbmittel.

Schließlich können die erfindungsgemäßen Formulierungen auch noch Komplexbildner wie EDTA, NTA, β-Alanindiessigsäure und Phosphonsäure, Farbstoffe zum Anfärben der kosmetischen Zubereitung, Trübungsmittel wie Latex, Styrol/PVP- und Styrol-Acrylamid-Copolymere, Ceramide, β-Glucane, Oligopepide, Hyaluronsäure, Perlglanzmittel wie Ethylenglykolmono- und -distearat und PEG-3-distearat, Pigmente, Lichtschutzmittel, Verdickungsmittel oder Treibmittel enthalten.

In einer bevorzugten Ausführungsform sind erfindungsgemäße Zusammensetzungen frei von Verbindungen ausgewählt aus Silikonverbindungen, Dipropylenglycol, Paraben und/oder Alkylparabene, Formaldehydabspaltern, wie Diazolidinyl Urea, Imidazolidinyl Urea und/oder DMDM Hydantoin, Konservierungsmittel auf Basis halogenorganischer Verbindungen wie z.B. Triclosan und Mischungen daraus. Besonders bevorzugt sind erfindungsgemäße Zusammensetzungen frei von Paraben und/oder Alkylparabenen, insbesondere bevorzugt, frei von Paraben und Alkylparabenen. Unter die Bezeichnung Paraben und Alkylparabene, fallen erfindungsgemäß insbesondere also 4-Hydroxybenzoesäure und 4-Hydroxybenzoesäureester, wie beispielsweise Methyl-4-hydroxybenzoat, Ethyl-4-hydroxybenzoat, Propyl-4-hydroxybenzoat und/oder Butyl-4-hydroxybenzoat. Weiterhin besonders bevorzugt sind erfindungsgemäße Zusammensetzungen frei von Silikonverbindungen. Dies kann insbesondere dann erwünscht sein, wenn die Zusammensetzungen im Arbeitsumfeld mit empfindlichen Oberflächen, wie beispielsweise Zu lackierende Oberflächenn, zur Anwendung kommen, da Silikonverbindungen möglicherweise Rückstände auf Werkstücken oder Produkten, hinterlassen könnten, die bei der Weiterverarbeitung negative Auswirkung auf das Produkt haben könnten. In einer speziell bevorzugten Ausführungsform können erfindungsgemäße Zusammensetzungen frei von Silikonverbindungen und frei von Paraben und frei von Alkylparabenen sein.

Die erfindungsgemäßen Zusammensetzungen können auf beliebige Weise hergestellt werden. Vorzugsweise sind erfindungsgemäße Zusammensetzungen erhältlich nach dem erfindungsgemäßen Verfahren gemäß Anspruch 12. Die erste Phase der Zusammensetzung wird in Schritt i) des Herstellungsverfahrens auf eine Temperatur von mehr als 65 °C, bevorzugt von mehr als 70 °C, insbesondere von 75 - 80 °C erhitzt.

Die zweite Phase der Zusammensetzung wird in Schritt ii) des Herstellungsverfahrens auf eine Temperatur von mehr als 60 °C, bevorzugt von mehr als 65 °C, insbesondere von 70 - 75 °C erhitzt.

Die Temperatur in Schritt v) des Herstellungsverfahrens beträgt bevorzugt weniger als 65 °C, insbesondere 60 °C.

Die Temperatur in Schritt vi) des Herstellungsverfahrens beträgt bevorzugt weniger als 60 °C, besonders bevorzugt weniger als 45 °C und insbesondere 40 °C.

Bevorzugt wird in den Schritten iv), v) und vii) des Herstellungsverfahrens jeweils 2 bis 20 Minuten gerührt, bevorzugt mindestens 5 Minuten.

Durch Anpassung der entsprechenden Mengen und Einsatz der entsprechenden Wirkstoffe, kann jede erfindungsgemäße Zusammensetzung durch dieses Verfahren hergestellt werden.

Erfindungsgemäße oder erfindungsgemäß hergestellte Zusammensetzungen werden bevorzugt als Creme, Lotion oder Gel auf der menschlichen Haut appliziert. Ein weiterer Gegenstand der vorliegenden Erfindung ist daher die Verwendung erfindungsgemäßer Zusammensetzungen zur topischen Anwendung auf der Haut. Bevorzugt kann jede Hautpartie wie z.B. Gesicht, Hände, Unterarme und der gesamte menschliche Körper mit erfindungsgemäßen Zusammensetzungen behandelt werden. Bevorzugt werden Hände Unterarme und Gesicht, insbesondere Hände und Unterarme, mit erfindungsgemäßen Zusammensetzungen behandelt. Dabei ist jede mögliche Form der Applikation von Zusammensetzungen auf die Haut erfindungsgemäß umfasst. Beispiele für mögliche Applikationen sind Auftragen mit Händen und mit Hilfsmitteln, Eincremen, Einreiben, Einsprühen, Einmassieren.

Ein weiterer Gegenstand der vorliegenden Erfindung ist daher die Verwendung erfindungsgemäßer Zusammensetzungen als Hautcreme insbesondere als Creme für Hände und Unterarme. Hierin ebenfalls offenbart wird die kosmetische Verwendung erfindungsgemäßer Zusammensetzungen zur Pflege der Haut.

Ein weiterer Gegenstand der vorliegenden Erfindung ist die kosmetische Verwendung erfindungsgemäßer Zusammensetzungen als Feuchtigkeitsspender für die Haut. Bevorzugt kann die kosmetische Verwendung erfindungsgemäßer Zusammensetzungen als langanhaltender Feuchtigkeitsspender für die Haut sein. Ein langanhaltender Feuchtigkeitsspender für die Haut kann erfindungsgemäß dann vorliegen, wenn Hautfeuchtigkeitsmessungen, wie insbesondere gemäß Beispiel 4, den feuchtigkeitsspendenden Effekt bestätigen. Hierin ebenfalls offenbart wird die kosmetische Verwendung erfindungsgemäßer Zusammensetzungen zur Reduktion eines rauen, trockenen und/oder angespannten Hautgefühls. Eine Reduktion eines rauen, trockenen und/oder angespannten Hautgefühls kann erfindungsgemäß dann vorliegen, wenn das subjektive Empfinden der entsprechenden Testperson dies bestätigt.

Insbesondere im Arbeitsalltag wird die Haut durch Umwelteinflüsse und arbeitsbedingte Einflüsse stark beansprucht. Ein weiterer Gegenstand der vorliegenden Erfindung ist daher die kosmetische Verwendung erfindungsgemäßer Zusammensetzungen auf beanspruchter Haut. Ein bevorzugter Gegenstand der vorliegenden Erfindung ist weiterhin die kosmetische Verwendung erfindungsgemäßer Zusammensetzungen zum Schutz von beanspruchter Haut. Hierin ebenfalls offenbart wird die kosmetische Verwendung erfindungsgemäßer Zusammensetzungen auf normaler Haut. Hierin ebenfalls offenbart wird die kosmetische Verwendung erfindungsgemäßer Zusammensetzungen zur täglichen Anwendung. Hierin ebenfalls offenbart wird die kosmetische Verwendung erfindungsgemäßer Zusammensetzungen zur täglichen Anwendung bei normaler beruflicher Beanspruchung. Unter normaler beruflicher Beanspruchung werden im Sinne dieser Erfindung beispielsweise Bürotätigkeit, Produktion, Vertrieb, handwerkliche Tätigkeit, einfache Labortätigkeit, Außentätigkeit bei normalen klimatischen Bedingungen wie z.B. bei Temperaturen zwischen 0°C und 30°C, gelegentlicher Kontakt mit Wasser oder Reinigungsmitteln, das gelegentliche Tragen von Handschuhen, welches zu Hauterweichungen führen kann, und alle alltäglichen, beruflichen Arbeiten, bei denen normale Einflüsse, im Gegensatz zu extremen Einflüssen wie beispielsweise, extreme Kälte deutlich unter 0°C, extreme Hitze, deutlich über 40°C, permanenter Hautkontakt mit Chemikalien, wie Formaldehyd, chemischen Wirkstoffen oder Reinigungsmitteln oder Belastung durch UV-Strahlung, auf die Haut einwirken, verstanden.

Selbstverständlich können die genannten Verwendungen überschneidend oder auch gleichzeitig auftreten. Eine bevorzugte Verwendung kann daher die, bevorzugt tägliche, Verwendung erfindungsgemäßer Zusammensetzungen als Hautcreme zur topischen Anwendung auf der Haut, insbesondere als Creme für Hände und Unterarme, auf beanspruchter oder normaler Haut zur Anwendung bei normaler beruflicher Beanspruchung und/oder zur Pflege und/oder als Feuchtigkeitsspender, insbesondere langanhaltender Feuchtigkeitsspender, für die Haut und/oder zum Schutz von beanspruchter Haut und/oder zur Reduktion eines rauen, trockenen und/oder angespannten Hautgefühls sein.

### Beispiel 1: Herstellung der Zusammensetzungen

Die in Tabelle 1 angeführten Zusammensetzungen werden gemäß dem beschriebenen Herstellungsverfahren hergestellt:
i) Herstellen einer ersten Phase durch Mischen der Wirkstoffe a), c), einem ersten Teil von d), so wie e) und Erhitzen der Phase auf eine Temperatur von mehr als 65 °C unter Rühren
ii) Herstellen einer zweiten Phase durch Mischen von mindestens einem der unter f) genannten Wirkstoffe mit Wasser und Erhitzen auf mehr als 60 °C unter Rühren
iii) Herstellen einer dritten Phase durch Mischen der Wirkstoffe b) und dem restlichen Teil von d) und Erhitzen der Phase auf mehr als 65 °C unter Rühren
iv) Homogenisieren der ersten und der zweiten Phase durch rühren bei einer Temperatur größer 60 °C
v) Mischen der in Schritt iv) erhaltenen Zusammensetzung mit der dritten Phase bei einer Temperatur kleiner 65 °C
vi) gegebenenfalls Zugabe von Parfüm, Konservierungsmittel, Betain, Urea und/oder weiteren Stoffen zu der in Schritt v) erhaltenen Zusammensetzung bei einer Temperatur kleiner 60 °C, bevorzugt kleiner 45°C, unter Rühren
vii) Einstellen des pH-Wertes unter Rühren.
viii) Entlüften der Zusammensetzung bei Raumtemperaturmit den Wirkstoffen a) bis f) wie in der Beschreibung definiert und in Tabelle 1 näher definiert.

Bei den Zusammensetzungen E1 und E2 handelt es sich um erfindungsgemäße Cremes. Die Vergleichsversuche V1 bis V10 zeigen Zusammensetzungen, die nicht unter den Erfindungsgegenstand fallen.

### Beispiel 2: Stabilitätstests

Die unterschiedlichen Zusammensetzungen wurden zunächst auf ihre Stabilität hin beurteilt. Dazu wurden die zu testenden Zusammensetzungen über einen Zeitraum von 3 Monaten jeweils bei Temperaturen von 4 °C, 20 bis 25 °C (Raumtemperatur) so wie bei 40 °C gelagert und anschließend auf ihre Konsistenz überprüft. Die Stabilität wurde auf Basis der folgenden Kriterien beurteilt:

| | |
|---|---|
| Stabil: | keine sichtbare Phasentrennung, kein Ausfallen von Feststoffen, unveränderte Konsistenz, unveränderte Farbe, unveränderter Geruch |
| wenig stabil: | leicht sichtbare Phasentrennung, teilweises Ausfallen von Feststoffen, leicht veränderte Konsistenz, geringfügige Veränderung der Farbe, schwache Veränderung des Geruchs, |
| instabil: | deutlich sichtbare Phasentrennung, sichtbares Ausfallen von Feststoffen, deutlich veränderte Konsistenz, deutliche Farbveränderung, deutliche Geruchsveränderung bis hin zum Empfinden eines schlechten Geruchs |

Die Ergebnisse des Stabilitätstests sind in Tabelle 1 aufgelistet.

### Beispiel 3: Subjektive Beurteilung Effektivität und Wirksamkeit

Die unterschiedlichen Zusammensetzungen wurden anschließend durch ein Testkollektiv aus 15 hautgesunden Männern und Frauen nach ihrem Aufziehverhalten und ihren pflegenden Eigenschaften bei dermaler Applikation auf den volaren Unterarm und auf die Hände beurteilt. Die Ergebnisse sind ebenfalls in Tabelle 1 angegeben.

Die Produkte wurden je nach ihrem Einziehverhalten in die Kategorien sehr gut, gut und schlecht eingestuft.

Die pflegenden Eigenschaften wurden zunächst nach einem positiven, neutralen bzw. negativen Eindruck eingeteilt und anschließend der wahrgenommene Effekt ermittelt. Folgende Eindrücke wurden mehrheitlich angeführt: starker Pflegeeindruck, kein Pflegeeindruck, wässriger Eindruck des Produkts (der oftmals durch ein Brechen der Zusammensetzung während des Auftragens hervorgerufen wird), okklusiver Eindruck des Produkts.

Alle Angaben beziehen sich, soweit nichts anderes vermerkt, auf Gewichtsprozent bezogen auf das Gesamtgewicht der Zusammensetzung. Ebenso sind alle angegebenen Verhältnisse als Gewichtsverhältnisse der jeweiligen Wirkstoffe zu verstehen.

**Tabelle 1 - Teil 1**

| | **E1** | **E2** | **V1** | **V2** | **V3** | **V4** |
|---|---|---|---|---|---|---|
| Isoamylcocoat | 4,5 | 4,23 | 4,5 | 4,5 | 4,5 | 4,5 |
| Glycerin | 3,0 | 3,4 | 3,0 | 3,0 | 3,0 | 3,0 |
| Caprylic/Capric Triglyceride | 3,5 | 3,25 | 3,5 | 3,5 | 3,5 | 3,5 |
| Polyglyceryl-3 Dicitrat/Stearat | 2,5 | 2,5 | 2,5 | 2,5 | 2,5 | 2,5 |
| Myristyl Myristat | 1,0 | 1,0 | 1,0 | 1,0 | 1,0 | 1,0 |
| Allantoin | 0,5 | 0,5 | 0,5 | 0,5 | 0,5 | 0,5 |
| Creatin | 0,5 | 0,5 | 0,5 | 0,5 | 0,5 | 0,5 |
| Stearylalkohol | 0,3 | 0,25 | 0,3 | 0,3 | 0,3 | 0,8 |
| Hydriertes Rizinusöl (HYDROGENATED CASTOR OIL) | 0,25 | 0,2 | 0,8 | - | 0,25 | 0,25 |
| Cera Microcristallina/ Paraffin | 0,25 | 0,2 | 0,2 | 0,18 | 0,25 | 0,25 |
| Acrylates/C10-30 Alkylacrylates Crosspolymer | 0,5 | 0,4 | 0,5 | 0,5 | 0,15 | 0,5 |
| Parfüm, Konservierungsmittel, pH Stellmittel, Urea, Betain, weitere Inhaltsstoffe | 0,5-3 | 0,5-3 | 0,5-3 | 0,5-3 | 0,5-3 | 0,5-3 |
| Wasser | a.d. 100 | a.d. 100 | a.d. 100 | a.d. 100 | a.d. 100 | a.d. 100 |
| | | | | | | |
| Verhältnis Glycerin zu Allantoin und Creatin | 3,3:1 | 3,4:1 | 3,3:1 | 3,3:1 | 3,3:1 | 3,3:1 |
| Verhältnis Polyglyceryl-3 Dicitrat/Stearat zu Urea | 125:1 | 125:1 | 125:1 | 125:1 | 125:1 | 125:1 |
| Verhältnis Betain zu Urea | 2:1 | 2:1 | 2:1 | 2:1 | 2:1 | 2:1 |
| Verhältnis chemisch modifizierte Wachskörper zu tierischen/pflanzlichen Wachskörpern | 1:1 | 1:1 | 4:1 | - | 1:1 | 1:1 |
| Gesamtverhältnis Wachskörper a) zu Polymer b) | 1:1 | 1:1 | 2,5:1 | 1:2,2 | 1:1 | 1:1 |
| Einziehverhalten | sehr gut | sehr gut | schlecht | gut | gut | schlecht |
| Stabilität der Zusammensetzung | stabil | stabil | stabil | instabil | instabil | wenig stabil |
| Eindruck auf der Haut/Haptik | positiv/ starker Pflegeeindruck | positiv/ starker Pflegeeindruck | negativ/ okklusiver Eindruck des Produkts | negativ/ wässriger Eindruck des Produkts niedrige Viskosität | neutral/ wässriger Eindruck, guter Pflegeeindruck | negativ/ okklusiver Eindruck des Produkts |

**Tabelle 1 - Teil 2**

| | **V5** | **V6** | **V7** | **V8** | **V9** | **V10** |
|---|---|---|---|---|---|---|
| Isoamylcocoat | 4,5 | 6,5 | 2,5 | 4,5 | 4,5 | 4,5 |
| Glycerin | 3,0 | 3,0 | 3,0 | 3,0 | 3,0 | 4,2 |
| Caprylic/Capric Triglyceride | 3,5 | 6,5 | 2,5 | 3,5 | 3,5 | 3,5 |
| Polyglyceryl-3 Dicitrat/Stearat | 2,5 | 2,5 | 2,5 | 3,0 | 3,0 | 3,0 |
| Myristyl Myristat | 1,0 | 1,0 | 1,0 | 1,6 | 0,4 | 1,0 |
| Allantoin | 0,6 | 0,6 | 0,6 | 0,6 | 0,6 | 0,6 |
| Creatin | 0,6 | 0,6 | 0,6 | 0,6 | 0,6 | - |
| Stearylalkohol | 0,08 | 0,3 | 0,3 | 0,3 | 0,3 | 0,3 |
| Hydriertes Rizinusöl (HYDROGENATED CASTOR OIL) | 0,25 | 0,25 | 0,25 | 0,25 | 0,25 | 0,25 |
| Cera Microcristallina/ Paraffin | 0,25 | 0,25 | 0,25 | 0,25 | 0,25 | 0,25 |
| Acrylates/C10-30 Alkylacrylates Crosspolymer | 0,5 | 0,5 | 0,5 | 0,5 | 0,5 | 0,5 |
| Parfüm, Konservierungsmittel, pH Stellmittel, Urea, Betain, weitere Inhaltsstoffe | 0,5-3 | 0,5-3 | 0,5-3 | 0,5-3 | 0,5-3 | 0,5-3 |
| Wasser | a.d. 100 | a.d. 100 | a.d. 100 | a.d. 100 | a.d. 100 | a.d. 100 |
| | | | | | | |
| Verhältnis Glycerin zu Allantoin und Creatin | 3,3:1 | 3,3:1 | 3,3:1 | 3,3:1 | 3,3:1 | 7:1 |
| Verhältnis Polyglyceryl-3 Dicitrat/Stearat zu Urea | 125:1 | 125:1 | 125:1 | 125:1 | 125:1 | 125:1 |
| Verhältnis Betain zu Urea | 2:1 | 2:1 | 2:1 | 2:1 | 2:1 | 2:1 |
| Verhältnis chemisch modifizierte Wachskörper zu tierischen/pflanzlichen Wachskörpern | 1:1 | 1:1 | 1:1 | 1:1 | 1:1 | 1:1 |
| Gesamtverhältnis Wachskörper a) zu Polymer b) | 1:1 | 1:1 | 1:1 | 1:1 | 1:1 | 1:1 |
| Einziehverhalten | schlecht | schlecht | gut | schlecht | schlecht | gut |
| Stabilität der Zusammensetzung | wenig stabil | stabil | stabil | wenig stabil | wenig stabil | stabil |
| Eindruck auf der Haut/Haptik | negativ/ Kein Pflegeeindruck | negativ/ Kein Pflegeeindruck | negativ/ Kein Pflegeeindruck | negativ/ okklusiver Eindruck des Produkts | negativ/ Kein Pflegeeindruck | negativ/ Kein Pflegeeindruck |

Die Versuche zeigen eindeutig, dass ausschließlich mit erfindungsgemäßen Zusammensetzungen ein rundum zufriedenstellendes Ergebnis in Bezug auf Stabilität der Zusammensetzung und Eindruck auf der Haut sowie Einziehverhalten in die Haut erzielt werden kann. Das schnelle Einziehverhalten hat neben dem angenehmen, pflegenden, nicht klebrigen Empfinden durch die Testpersonen, den positiven Effekt, dass die Berührung von Oberflächen mit den mit erfindungsgemäßen Zusammensetzungen behandelten Hautpartien zu deutlich weniger unerwünschten Rückständen auf den entsprechenden Oberflächen, wie beispielsweise Plastikoberflächen, wie Tastatur und Telefon, Glasoberflächen, Plexiglas oder modifizierte Glasoberflächen, zu lackierende Oberflächen, Lacke, insbesondere Autolacke, Lasuren, Keramik, Porzellan, Metall, Legierungen, Labormaterialien oder mit Polymeren behandelten Oberflächen, führt als bei den nicht erfindungsgemäßen Zusammensetzungen.

### Beispiel 4: Effektivität und Wirksamkeit - feuchtigkeitsspendender Effekt

Neben dem subjektiven Eindruck der Testpersonen, wurde die erfidungsgemäße Creme E2 zudem auf ihre Effektivität und Wirksamkeit getestet. Hierzu wurden als messbare Parameter die Bestimmung des TEWL (Transepidermal Water Loss) und der Hautfeuchtigkeit ausgewählt. Der Vergleich erfolgte zwischen dem mit Creme applizierten Arm (dermale Applikation auf den volaren Unterarm) und dem nicht mit Creme applizierten Arm eines Testkollektivs aus 15 hautgesunden Männern und Frauen über einen Zeitraum von 14 Tagen.

Zur Bestimmung des Ausgangswertes des TEWL und der Hautfeuchtigkeit wurde mit Hilfe einer Schablone ein Testareal (ca. 4 x 4 cm) auf die Unterseite des rechten und linken Arms eines jeden Probanden markiert. Innerhalb dieses Testareals wurden Dreifachbestimmungen des TEWL und Fünffachbestimmungen der Hautfeuchtigkeit durchgeführt (Tag 0). Der TEWL wurde mit einem Multiprobe Adapter MPA 5 mit Tewameter® Sonde TM300 und die Hautfeuchtigkeit mit einem Multiprobe Adapter MPA 5 mit Corneometer® Sonde CM 825 bestimmt. Die Messungen wurden bei 20°C und 40 % rel Luftfeuchtigkeit durchgeführt.

Unmittelbar nach den Messungen erfolgt das Eincremen, wobei jeweils immer der linke Arm eines jeden Probanden morgens und abends mit der zu testenden Creme eingecremt wird, der rechte Arm während der gesamten Versuchsdauer frei von jeglicher Creme (Negativkontrolle) bleibt.

Weitere Bestimmungen des TEWL und der Hautfeuchtigkeit wie oben beschrieben erfolgen an Tag 7 und Tag 14, wobei das morgendliche Eincremen am Morgen des Messtages erst nach der Bestimmung des TEWL und der Hautfeuchtigkeit erfolgt, um nicht evtl. aus der Creme verdunstendes Wasser bei den Messungen zu erfassen.

Die Mittelwerte und die Standardabweichung aus den Messwerten des Testkollektivs aus 15 Personen und der jeweiligen Dreifachbestimmung (TEWL) bzw. Fünffachbestimmung (Hautfeuchtigkeit) des rechten bzw. des linken Arms eines jeden Probanden an Tag 0, Tag 7 und Tag 14 wurden berechnet. Die Ergebnisse des TEWL sind in Tabelle 2, die Ergebnisse der Hautfeuchtigkeitsmessung sind in Tabelle 3 angegeben:

**Tabelle 2: Ergebnisse TEWL**

| | Vor Applikation | Tag 7 | Tag 14 |
|---|---|---|---|
| Linker Arm | 7,4 ± 0,8 | 6,1 ± 0,8 | 6,1 ± 0,8 |
| Rechter Arm | 6,5 ± 0,8 | 6,0 ± 0,8 | 6,1 ± 0,8 |

**Tabelle 3: Ergebnisse Hautfeuchtigkeitsmessung**

| | Vor Applikation | Tag 7 | Tag 14 |
|---|---|---|---|
| Linker Arm | 31,9 ± 2 | 35,8 ± 2 | 38,1 ± 2 |
| Rechter Arm | 30,5 ± 2 | 29,7 ± 2 | 32,4 ± 2 |

Der TEWL bleibt nach 14 Tagen sowohl am rechten Arm (ohne Creme) als auch am linken Arm (mit Creme) im Bereich der Standardabweichung ohne nennenswerten Unterschied. Der Messwert der Hautfeuchtigkeit bleibt nach 14 Tagen am rechten Arm (ohne Creme) ebenfalls im Bereich der Standardabweichung ohne nennenswerten Unterschied.

Die deutliche Zunahme der Hautfeuchtigkeit von 19,4 % am linken Arm (mit Creme) im Vergleich zum rechten Arm zeigt klar die Wirksamkeit und die feuchtigkeitsspendende Wirkung der erfindungsgemäßen Zusammensetzung, wodurch insbesondere die gute Pflege der Haut bei Alltagseinflüssen entstammt. Daher eignen sich die erfindungsgemäßen Zusammensetzungen besonders gut zur täglichen Anwendung bei normaler alltäglicher Beanspruchung der Haut. Versuche mit weiteren erfindungsgemäßen Zusammensetzungen führen zu sehr ähnlichen Ergebnissen.

### Beispiel 5: Hautverträglichkeit

Die erfidungsgemäße Creme E2 wurde zudem auf ihre Hautverträglichkeit getestet. Der Test erfolgte mit einem Testkollektiv aus 21 hautgesunden Männern und Frauen. Zunächst wurde eine Testfläche pro Testsubstanz auf den Unterarmen gekennzeichnet und die Haut in diesen Testflächen durch Anritzen mittels einer sterilen Kanüle vorgeschädigt. Etwa 0,2 g der Testsubstanz wurde in eine Finn - Kammer gefüllt, auf die Testflächen eines Unterarmes appliziert und für 23 Stunden fixiert. Nach 23 Stunden erfolgte die Abnahme der Finn - Kammern und das Abspülen mit Leitungswasser durch den Probanden. Eine Stunde später erfolgte die Kontrolle (Visual Score) der Testfelder. Die Applikation wurde an drei aufeinander folgenden Tagen durchgeführt. Als Kontrolle wurde Vaseline DAB 10 wie oben beschrieben getestet.
Bewertung und Berechnung

Der Visual Score wird täglich 1 Stunde nach dem Entfernen der Kammern gemäß Tabelle 4 ermittelt.

**Tabelle 4: Visual Score**

| Symptome/Beurteilung der Skarifikation Punkte | Visual Score x |
|---|---|
| negativ | 0 |
| Rötung begrenzt auf Kratzer | 1 |
| breitere Banden, gesteigerte Rötung mit/ohne Bläschen, (klare Flüssigkeit), Pusteln (Eiter) oder Erosionen | 2 |
| starkes Erythem mit teilweisem Zusammenfluß mit/ohne weitere Läsionen | 3 |
| zusammenfließendes schweres Erythem, Ödem, Nekrose Blasenbildung | 4 |

Liegt eine Bewertung zwischen zwei Bewertungspunkten, ist zur weiteren Differenzierung die Beurteilung in 0,5er oder kleineren Schritten möglich.

Für die Bewertung der relativen Hautverträglichkeit der Testprodukte ist der Mittelwert der Visual Score Werte x mit Blick auf die mittlere Applikationszeit heranzuziehen. Diese Werte sind für E1 und den Standard Vaseline DAB 10 in Tabelle 5 angegeben.

**Tabelle 5: Ergebnisse Hautverträglichkeit**

| | Mittlere Visual Score-Werte x | Mittlere Applikationsdauer |
|---|---|---|
| Creme E1 | 0,4 | 63,5 h |
| Standard Vaseline | 0,5 | 63,5 h |

Versuche mit weiteren erfindungsgemäßen Zusammensetzungen führen zu sehr ähnlichen Ergebnissen. Diese Ergebnisse zeigen, dass erfindungsgemäße Zusammensetzungen äußerst hautfreundlich sind und zu keinerlei nennenswerten Hautirritationen führen. Bei gleicher Applikationszeit schneiden erfindungsgemäße Zusammensetzungen im Hautverträglichkeitstest genauso gut bzw. tendenziell leicht besser ab als der Standard Vaseline DAB 10.

Ähnliche Testprotokolle sind im Stand der Technik bekannt und beispielsweise unter P.J. Frosch, A.M. Kligman, Contact Dermatitis, 5, 73 (1979); P.J. Frosch, A.M. Kligman, J. Am. Acad. Dermatol., 1, 35 (1979) oder P.J. Frosch, A.M. Kligman, Contact Dermatitis, 2, 314 (1976) publiziert. Der Inhalt dieser Literaturstellen ist hier ganzheitlich mit einbezogen

## Patentansprüche

1. Kosmetische und/oder dermatologische Zusammensetzung **dadurch gekennzeichnet, dass** die Zusammensetzung als Wirkstoffkombination, bezogen auf das Gesamtgewicht der Zusammensetzung
a) 0,2 bis 0,8 Gew.-% mindestens eines Wachskörpers, ausgewähltaus aus Candelillawachs, Carnaubawachs, Japanwachs, Espartowachs, Korkwachs, Reiskeimölwachs, Guaramawachs, Ouricurywachs, Montanwachs, Jojobawachs, Arganienwachs, Meerrettichbaumwachs, Olivenwachs, Sheabutter, Beerenwachs, Bienenwachs, Traubenkernwachs, Macadamianusswachs, Sonnenblumenwachs, Baumwollsamenwachs, Schellackwachs, Walrat, Lanolin, Bürzelfett, Ceresin, Ozokerit (Erdwachs), Petrolatum, Paraffinwachse, mikrokristalline Wachse, Montanesterwachse, Sasolwachse, hydrierte Jojobwachse und hydriertes Pflanzenöl;
b) 0,3 bis 1 Gew.-% mindestens eines Polymers, basierend auf mindestens einem Acrylsäure-Monomer und/oder mindestens einem Ester eines Acrylsäure-Monomers;
c) 0,1 bis 0,75 Gew.-% mindestens eines linearen, gesättigten unsubstituierten Fettalkohols mit 12 bis 20 Kohlenstoffatomen;
d) 6 bis 12 Gew.-% mindestens eines Emollients ausgewählt aus PEG-30 Glyceryl Cocoat, PEG-6 Capryl/Capric Glycerid, Sorbitan Sesquicaprylat, Sucrose Cocoat, Isoamyl Cocoat, Polyglyceryl-3 Caprat, PPG-3 Myristyl Ether, PPG-11 Stearyl Ether, Cetearyl Isononanoate, Cetyl Ethylhexanoate, Caprylic / Capric Triglyceride, Diethylhexyl Carbonate, Decyl Oleate, PPG-15 Stearyl Ether, Octyldodecanol, Isocetyl Palmitat, Isohexadecane, Cetearyl Ethylhexanoat, Isopropyl Myristat, Oleyl Erucat, Ethylhexyl Palmitat, Ethylhexyl Stearat, Isopropyl Palmitat, PPG-14 Butyl Ether, Trüsostearin, C12-15 Alkylbenzoat, Cetyl Ricenoleate, Glyceryl Ricenoleat, Glyceryl Stearat, Isocetyl Palmitat, Isocetyl stearat, Tocopheryl Linoleat, Methylheptyl Isostearat und Mischungen daraus;
e) 0,5 bis 1,5 Gew.-% mindestens eines linearen, gesättigten, unsubstituierten Esters mit 20 bis 34 Kohlenstoffatomen aus einer linearen, gesättigten unsubstituierten Fettsäure und einem linearen, gesättigten, unsubstituierten Fettalkohol und
f) Glycerin und mindestens einen Wirkstoff ausgewählt aus Creatin und/oder Allantoin enthält, wobei das Gewichtsverhältnis des Gesamtgewichts von Creatin und/oder Allantoin zu dem Gesamtgewicht an Glycerin 1:6 bis 1:2.

2. Zusammensetzung nach Anspruch 1 enthaltend als Wirkstoffkombination, bezogen auf das Gesamtgewicht der Zusammensetzung
a) 0,3 bis 0,5 Gew.-% mindestens eines Wachskörpers ansgewählt aus Candelillawachs, Carnaubawachs, Japanwachs, Espartowachs, Korkwachs, Reiskeimölwachs, Guaramawachs, Ouricurywachs, Montanwachs, Jojobawachs, Arganienwachs, Meerrettichbaumwachs, Olivenwachs, Sheabutter, Beerenwachs, Bienenwachs, Traubenkernwachs, Macadamianusswachs, Sonnenblumenwachs, Baumwollsamenwachs, Schellackwachs, Walrat, Lanolin, Bürzelfett, Ceresin, Ozokerit (Erdwachs), Petrolatum, Paraffinwachse, mikrokristalline Wachse, Montanesterwachse, Sasolwachse, hydrierte Jojobwachse und hydriertes Pflanzenöl;
b) 0,35 bis 0,5 Gew.-% mindestens eines Polymers, basierend auf mindestens einem Acrylsäure-Monomer und/oder mindestens einem Ester eines Acrylsäure-Monomers;
c) 0,2 bis 0,3 Gew.-% mindestens eines linearen, gesättigten unsubstituierten Fettalkohols mit 12 bis 20 Kohlenstoffatomen;
d) 7 bis 10 Gew.-% mindestens eines Emollients, ausgewählt aus PEG-30 Glyceryl Cocoat, PEG-6 Capryl/Capric Glycerid, Sorbitan Sesquicaprylat, Sucrose Cocoat, Isoamyl Cocoat Polyglyceryl-3 Caprat, PPG-3 Myristyl Ether, PPG-11 Stearyl Ether, Cetearyl Isononanoate, Cetyl Ethylhexanoate, Caprylic / Capric Triglyceride, Decal Cocoate,Diethylhexyl Carbonate, Decyl Oleate, PPG-15 Stearyl Ether, Octyldodecanol, Isocetyl Palmitat, Isohexadecane, Cetearyl Ethylhexanoat, Isopropyl Myristat, Oleyl Erucat, Ethylhexyl Palmitat, Ethylhexyl Stearat, Isopropyl Palmitat, PPG-14 Butyl Ether, Trüsostearin, C12-15 Alkylbenzoat, Cetyl Ricenoleate, Glyceryl Ricenoleat, Glyceryl Stearat, Isocetyl Palmitat, Isocetyl stearat, Tocopheryl Linoleat, Methylheptyl Isostearat und Mischungen daraus;
e) 0,8 bis 1,2 Gew.-% mindestens eines Esters mit 20 bis 34 Kohlenstoffatomen aus einer linearen, gesättigten, unsubstituierten Fettsäure und einem linearen, gesättigten, unsubstituierten Fettalkohol und
f) 0,2 bis 8 Gew.-% Glycerin und mindestens eines Wirkstoffs ausgewählt aus Creatin und/oder Allantoin, wobei das Gewichtsverhältnis des Gesamtgewichts von Creatin und/oder Allantoin zu dem Gesamtgewicht an Glycerin 1:6 bis 1:2.

3. Zusammensetzung nach einem der Ansprüche 1 oder 2 enthaltend als Wirkstoffe a) bis f)
a) Mindestens einen tierischen und/oder pflanzlichen Wachskörper und mindestens einen chemisch modifizierten Wachskörper wobei das Gewichtsverhältnis des Gesamtgewichts des/der eingesetzten tierischen und pflanzlichen Wachskörper(s) zu dem Gesamtgewicht des/der eingesetzten chemisch modifizierten Wachskörper 1:2 bis 2:1, bevorzugt 1:1 beträgt;
b) mindestens eines Polymers, basierend auf mindestens einem Acrylsäure-Monomer und/oder mindestens einem Ester eines Acrylsäure-Monomers, ausgewählt aus Homopolymeren der Acrylsäure und/oder Copolymeren aus Acrylsäure und/oder Alkylester der Acrylsäure, bevorzugt C10 bis C30 Alkylester der Acrylsäure, und insbesondere bevorzugt Acrylates/C 10-30 Alkyl Acrylate Crosspolymer;
c) mindestens eines linearen, gesättigten unsubstituierten Fettalkohols mit 16 bis 18 Kohlenstoffatomen, ausgewählt aus Stearyl Alkohol und Cetyl Alkohol,
d) mindestens ein Emollient ausgewählt aus PEG-30 Glyceryl Cocoat, PEG-6 Capryl/Capric Glycerid, Sorbitan Sesquicaprylat, Sucrose Cocoat, Isoamyl Cocoat, Polyglyceryl-3 Caprat, PPG-3 Myristyl Ether, PPG-11 Stearyl Ether, Cetearyl Isononanoate, Cetyl Ethylhexanoate, Caprylic / Capric Triglyceride, Diethylhexyl Carbonate, Decyl Oleate, PPG-15 Stearyl Ether, Octyldodecanol, Isocetyl Palmitat, Isohexadecane, Cetearyl Ethylhexanoat, Isopropyl Myristat, Oleyl Erucat, Ethylhexyl Palmitat, Ethylhexyl Stearat, Isopropyl Palmitat, PPG-14 Butyl Ether, Trüsostearin, C12-15 Alkylbenzoat, Cetyl Ricenoleate, Glyceryl Ricenoleat, Glyceryl Stearat, Isocetyl Palmitat, Isocetyl stearat, Tocopheryl Linoleat, Methylheptyl Isostearat und Mischungen daraus, insbesondere bevorzugt Isoamyl Cocoat;
e) mindestens einen Ester mit 24 bis 32, bevorzugt mit 28, Kohlenstoffatomen, aus einer linearen, gesättigten, unsubstituierten Fettsäure und einem linearen, gesättigten, unsubstituierten Fettalkohol, wobei sich die Anzahl der Kohlenstoffatome in der Fettsäurekomponente und die Anzahl der Kohlenstoffatome in der Fettalkoholkomponente um nicht mehr als 2, bevorzugt um nicht mehr als 1, besonders bevorzugt um kein Kohlenstoffatom(e) unterscheidet, insbesondere bevorzugt ausgewählt aus Cetyl Palmitat, Cetyl Stearat, Myristyl Myristat und Mischungen daraus und
f) Glycerin und mindestens einen Wirkstoff ausgewählt aus Creatin und/oder Allantoin, wobei das Gewichtsverhältnis des Gesamtgewichts von Creatin und/oder Allantoin zu dem Gesamtgewicht an Glycerin 1:6 bis 1:2, wobei die Zusammensetzung keine weiteren Stoffe enthält, die in die in den Gruppen a) bis f) genannten Wirkstoffgruppen fallen.

4. Zusammensetzung nach einem der Ansprüche 1 bis 3, **dadurch gekennzeichnet, dass** die Zusammensetzung als Komponente f) Creatin, Allantoin und Glycerin enthält.

5. Zusammensetzung nach einem der Ansprüche 1 bis 4, wobei die Zusammensetzung als Komponente f) Glycerin und mindestens einen weiteren Wirkstoff ausgewählt aus Creatin und/oder Allantoin enthält, **dadurch gekennzeichnet, dass** das Gewichtsverhältnis des Gesamtgewichts von Creatin und Allantoin zu dem Gesamtgewicht an Glycerin 1:3,2 bis 1:3,6 beträgt.

6. Zusammensetzung nach einem der Ansprüche 1 bis 5 **dadurch gekennzeichnet, dass** das Gewichtsverhältnis des Gesamtgewichts an Wachskomponente a) zu dem Gesamtgewicht an Polymer der Komponente b) 2:1 bis 1:2, bevorzugt 1:1 beträgt.

7. Zusammensetzung nach einem der Ansprüche 1 bis 6, **dadurch gekennzeichnet, dass** die Zusammensetzung weiterhin die Wirkstoffe Polyglyceryl-3-dicitrate/stearate und Urea enthält.

8. Zusammensetzung nach Anspruch 7 **dadurch gekennzeichnet, dass** Polyglyceryl-3-dicitrate/stearate und Urea in einem Gewichtsverhältnis von 100:1 bis 150:1, bevorzugt von 125:1 eingesetzt werden.

9. Zusammensetzung nach einem der Ansprüche 1 bis 8, weiterhin enthaltend die Wirkstoffe Betain und Urea **dadurch gekennzeichnet, dass** Betain und Urea in einem Gewichtsverhältnis von 1:4 bis 1:6 eingesetzt werden.

10. Zusammensetzung nach einem der Ansprüche 1 bis 9, **dadurch gekennzeichnet, dass** die Zusammensetzung weiterhin mindestens 70 Gew.-%, bevorzugt mindestens 80 Gew.-% Wasser enthält.

11. Zusammensetzung nach einem der Ansprüche 1 bis 10, **dadurch gekennzeichnet, dass** die Zusammensetzung frei von Silikonverbindungen und/oder frei von Paraben und/oder frei von Alkylparabenen ist.

12. Verfahren zur Herstellung von Zusammensetzungen nach einem der Ansprüche 1 bis 11 umfassend die Schritte
i) Herstellen einer ersten Phase durch Mischen der Wirkstoffe a), c), einem ersten Teil von d), so wie e);
ii) Herstellen einer zweiten Phase durch Mischen von mindestens einem der unter f) genannten Wirkstoffe mit Wasser;
iii) Herstellen einer dritten Phase durch Mischen der Wirkstoffe b) und dem restlichen Teil von d);
iv) Homogenisieren der ersten und der zweiten Phase durch rühren;
v) Mischen der in Schritt iv) erhaltenen Zusammensetzung mit der dritten Phase;
vi) gegebenenfalls Zugabe von Parfüm, Konservierungsmittel, Betain, Urea und/oder weiteren Stoffen zu der in Schritt v) erhaltenen Zusammensetzung und
vii) Einstellen des pH-Wertes und
viii) gegebenenfalls Entlüften der Zusammensetzung bei Raumtemperatur.

13. Verwendung von Zusammensetzungen nach einem der Ansprüche 1 bis 11 als Hautcreme insbesondere als Creme für Hände und Unterarme.

14. Kosmetische Verwendung von Zusammensetzungen nach einem der Ansprüche 1 bis 11 als Feuchtigkeitsspender, insbesondere langanhaltender Feuchtigkeitsspender, für die Haut.

15. Kosmetische Verwendung von Zusammensetzungen nach einem der Ansprüche 1 bis 11 zum Schutz von beanspruchter Haut.

## Claims

1. Cosmetic and/or dermatological composition, **characterised in that** the composition contains as an active ingredient combination, based on the total weight of the composition,
a) 0.2 to 0.8 wt.% of at least one wax body, selected from candelilla wax, carnauba wax, Japan wax, esparto wax, cork wax, rice germ oil wax, guarama wax, ouricury wax, montan wax, jojoba wax, argan wax, horseradish tree wax, olive wax, shea butter, berry wax, beeswax, grape seed wax, macadamia nut wax, sunflower wax, cotton seed wax, shellac wax, spermaceti, lanolin, rump fat, ceresin, ozocerite (earth wax), petrolatum, paraffin waxes, microcrystalline waxes, montan ester waxes, Sasol waxes, hydrogenated jojoba waxes and hydrogenated vegetable oil;
b) 0.3 to 1 wt.% of at least one polymer based on at least one acrylic acid monomer and/or at least one ester of an acrylic acid monomer;
c) 0.1 to 0.75 wt.% of at least one linear, saturated, unsubstituted fatty alcohol having 12 to 20 carbon atoms;
d) 6 to 12 wt.% of at least one emollient, selected from PEG-30 glyceryl cocoate, PEG-6 caprylic/capric glyceride, sorbitan sesquicaprylate, sucrose cocoate, isoamyl cocoate, polyglyceryl-3 caprate, PPG-3 myristyl ether, PPG-11 stearyl ether, cetearyl isononanoate, cetyl ethylhexanoate, caprylic/capric triglycerides, diethylhexyl carbonate, decyl oleate, PPG-15 stearyl ether, octyldodecanol, isocetyl palmitate, isohexadecane, cetearyl ethylhexanoate, isopropyl myristate, oleyl erucate, ethylhexyl palmitate, ethylhexyl stearate, isopropyl palmitate, PPG-14 butyl ether, triisostearin, C12-15 alkyl benzoate, cetyl ricinoleate, glyceryl ricinoleate, glyceryl stearate, isocetyl palmitate, isocetyl stearate, tocopheryl linoleate, methylheptyl isostearate, and mixtures thereof;
e) 0.5 to 1.5 wt.% of at least one linear, saturated, unsubstituted ester having 20 to 34 carbon atoms of a linear, saturated, unsubstituted fatty acid and a linear, saturated, unsubstituted fatty alcohol; and
f) glycerol and at least one active ingredient selected from creatine and/or allantoin, wherein the weight ratio of the total weight of creatine and/or allantoin to the total weight of glycerol is from 1:6 to 1:2.

2. Composition according to claim 1, containing as an active ingredient combination, based on the total weight of the composition
a) 0.3 to 0.5 wt.% of at least one wax body, selected from candelilla wax, carnauba wax, Japan wax, esparto wax, cork wax, rice germ oil wax, guarama wax, ouricury wax, montan wax, jojoba wax, argan wax, horseradish tree wax, olive wax, shea butter, berry wax, beeswax, grape seed wax, macadamia nut wax, sunflower wax, cotton seed wax, shellac wax, spermaceti, lanolin, rump fat, ceresin, ozocerite (earth wax), petrolatum, paraffin waxes, microcrystalline waxes, montan ester waxes, Sasol waxes, hydrogenated jojoba waxes and hydrogenated vegetable oil;
b) 0.35 to 0.5 wt.% of at least one polymer based on at least one acrylic acid monomer and/or at least one ester of an acrylic acid monomer;
c) 0.2 to 0.3 wt.% of at least one linear, saturated, unsubstituted fatty alcohol having 12 to 20 carbon atoms;
d) 7 to 10 wt.% of at least one emollient, selected from PEG-30 glyceryl cocoate, PEG-6 caprylic/capric glyceride, sorbitan sesquicaprylate, sucrose cocoate, isoamyl cocoate, polyglyceryl-3 caprate, PPG-3 myristyl ether, PPG-11 stearyl ether, cetearyl isononanoate, cetyl ethylhexanoate, caprylic/capric triglycerides, decyl cocoate, diethylhexyl carbonate, decyl oleate, PPG-15 stearyl ether, octyldodecanol, isocetyl palmitate, isohexadecane, cetearyl ethylhexanoate, isopropyl myristate, oleyl erucate, ethylhexyl palmitate, ethylhexyl stearate, isopropyl palmitate, PPG-14 butyl ether, triisostearin, C12-15 alkyl benzoate, cetyl ricinoleate, glyceryl ricinoleate, glyceryl stearate, isocetyl palmitate, isocetyl stearate, tocopheryl linoleate, methylheptyl isostearate, and mixtures thereof;
e) 0.8 to 1.2 wt.% of at least one ester having 20 to 34 carbon atoms from a linear, saturated, unsubstituted fatty acid and a linear, saturated, unsubstituted fatty alcohol; and
f) 0.2 to 8 wt.% of glycerol and at least one active ingredient selected from creatine and/or allantoin, wherein the weight ratio of the total weight of creatine and/or allantoin to the total weight of glycerol is from 1:6 to 1:2.

3. Composition according to either claim 1 or claim 2, containing as active ingredients a) to f)
a) at least one animal and/or vegetable wax body and at least one chemically modified wax body, wherein the weight ratio of the total weight of the animal and vegetable wax body or bodies used to the total weight of the chemically modified wax body or bodies used is from 1:2 to 2:1, preferably 1:1;
b) at least one polymer based on at least one acrylic acid monomer and/or at least one ester of an acrylic acid monomer, selected from homopolymers of acrylic acid and/or copolymers of acrylic acid and/or alkyl esters of acrylic acid, preferably C10 to C30 alkyl esters of acrylic acid, and particularly preferably acrylates/C10-30 alkyl acrylate cross polymer;
c) at least one linear, saturated, unsubstituted fatty alcohol having 16 to 18 carbon atoms, selected from stearyl alcohol and cetyl alcohol;
d) at least one emollient, selected from PEG-30 glyceryl cocoate, PEG-6 caprylic/capric glyceride, sorbitan sesquicaprylate, sucrose cocoate, isoamyl cocoate, polyglyceryl-3 caprate, PPG-3 myristyl ether, PPG-11 stearyl ether, cetearyl isononanoate, cetyl ethylhexanoate, caprylic/capric triglycerides, diethylhexyl carbonate, decyl oleate, PPG-15 stearyl ether, octyldodecanol, isocetyl palmitate, isohexadecane, cetearyl ethylhexanoate, isopropyl myristate, oleyl erucate, ethylhexyl palmitate, ethylhexyl stearate, isopropyl palmitate, PPG-14 butyl ether, triisostearin, C12-15 alkyl benzoate, cetyl ricinoleate, glyceryl ricinoleate, glyceryl stearate, isocetyl palmitate, isocetyl stearate, tocopheryl linoleate, methylheptyl isostearate, and mixtures thereof, particularly preferably isoamyl cocoate;
e) at least one ester having 24 to 32, preferably 28, carbon atoms from a linear, saturated, unsubstituted fatty acid and a linear, saturated, unsubstituted fatty alcohol, wherein the number of carbon atoms in the fatty acid component and the number of carbon atoms in the fatty alcohol component differ by no more than 2, preferably by no more than 1, particularly preferably by no, carbon atom(s), particularly preferably selected from cetyl palmitate, cetyl stearate, myristyl myristate, and mixtures thereof; and
f) glycerol and at least one active ingredient selected from creatine and/or allantoin, wherein the weight ratio of the total weight of creatine and/or allantoin to the total weight of glycerol is from 1:6 to 1:2, wherein the composition does not contain any other substances which are included in the active ingredient groups mentioned in the groups a) to f).

4. Composition according to any one of claims 1 to 3, **characterised in that** the composition contains creatine, allantoin and glycerol as component f).

5. Composition according to any one of claims 1 to 4, wherein the composition contains glycerol and at least one further active ingredient selected from creatine and/or allantoin as component f), **characterised in that** the weight ratio of the total weight of creatine and allantoin to the total weight of glycerol is from 1:3.2 to 1:3.6.

6. Composition according to any one of claims 1 to 5, **characterised in that** the weight ratio of the total weight of wax component a) to the total weight of the polymer of component b) is from 2:1 to 1:2, preferably 1:1.

7. Composition according to any one of claims 1 to 6, **characterised in that** the composition further contains the active ingredients polyglyceryl-3-dicitrate/stearate and urea.

8. Composition according to claim 7, **characterised in that** polyglyceryl-3 dicitrate/stearate and urea are used in a weight ratio of from 100:1 to 150:1, preferably of 125:1.

9. Composition according to any one of claims 1 to 8, further containing the active ingredients betaine and urea, **characterised in that** betaine and urea are used in a weight ratio of from 1:4 to 1:6.

10. Composition according to any one of claims 1 to 9, **characterised in that** the composition further contains at least 70 wt.%, preferably at least 80 wt.%, water.

11. Composition according to any one of claims 1 to 10, **characterised in that** the composition is free of silicone compounds and/or free of paraben and/or free of alkyl parabens.

12. Method for preparing compositions according to any one of claims 1 to 11, comprising the steps of
i) preparing a first phase by mixing the active ingredients a), c), a first part of d), and e);
ii) preparing a second phase by mixing at least one of the active ingredients mentioned in f) with water;
iii) preparing a third phase by mixing the active ingredients b) and the remaining part of d);
iv) homogenising the first and the second phase by stirring;
v) mixing the composition obtained in step iv) with the third phase;
vi) optionally adding perfume, preservative, betaine, urea and/or other substances to the composition obtained in step v); and
vii) adjusting the pH; and
viii) optionally deaerating the composition at room temperature.

13. Use of compositions according to any one of claims 1 to 11 as a skin cream, in particular as a cream for the hands and forearms.

14. Cosmetic use of compositions according to any one of claims 1 to 11 as a moisturiser, in particular a long-lasting moisturiser, for the skin.

15. Cosmetic use of compositions according to any one of claims 1 to 11 for protecting stressed skin.

## Revendications

1. Composition cosmétique et/ou dermatologique, **caractérisée en ce que** la composition contient en tant que combinaison de principes actifs, par rapport au poids total de la composition
a) 0,2 à 0,8 % en poids d'au moins un corps de cire choisi parmi la cire de candelilla, la cire de carnauba, la cire du Japon, la cire de sparte, la cire de liège, la cire d'huile de germes de riz, la cire de guarana, la cire d'Ouricuri, la cire de lignite, la cire de jojoba, la cire d'arganier, la cire de moringa oleifera, la cire d'olive, le beurre de karité, la cire de baies, la cire d'abeille, la cire de pépin de raison, la cire de noix de macadamia, la cire de tournesol, la cire de graine de coton, la cire de gomme-laque, le spermaceti, la lanoline, la graisse de Bürzel, la cérésine, l'ozokérite (cire naturelle), la vaseline, les cires de paraffine, les cires microcristallines, les cires d'ester de lignite, les cires de Sasol, les cires de jojoba hydrogénées et l'huile végétale hydrogénée ;
b) 0,3 à 1 % en poids d'au moins un polymère à base d'au moins un monomère d'acide acrylique et/ou d'au moins un ester d'un monomère d'acide acrylique ;
c) 0,1 à 0,75 % en poids d'au moins un alcool gras linéaire saturé non substitué comprenant 12 à 20 atomes de carbone ;
d) 6 à 12 % en poids d'au moins un émollient choisi parmi le PEG-30 glyceryl cocoate, le PEG-6 capryl/capric glycéride, le sorbitan sesquicaprylate, le sucrose cocoate, l'isomyl cocoate, le polyglyceryl-3 caprate, le PPG-3 myristyl éther, le PPG-11 stearyl éther, le cetearyl isononanoate, le cethyl éthylhexanoate, les caprylic/capric triglycérides, le diethylhexyl carbonate, le decyl oléate, le PPG-15 stearyl éther, l'octyldodécanol, l'isocetyl palmitate, l'isohexadécane, le cetearyl éthylhexanoate, l'isopropyl myristate, l'oleyl erucate, l'ethylhexyl palmitate, l'ethylhexyl stéarate, l'isopropyl palmitate, le PPG-14 butyl éther, la triisostéarine, le C12-15 alkylbenzoate, le cetyl ricénoleate, le glyceryl ricenoléate, le glyceryl stéarate, l'isocetyl palmitate, l'isocetyl stéarate, le tocopheryl linoléate, le methylheptyl isostéarate et des mélanges de ces derniers ;
e) 0,5 à 1,5 % en poids d'au moins un ester linéaire, saturé non substitué comprenant 20 à 34 atomes de carbone d'un acide gras linéaire, saturé non substitué et d'un alcool gras linéaire, saturé, non substitué ; et
f) de la glycérine et au moins un principe actif choisi parmi la créatine et/ou l'allantoïne, dans laquelle le rapport de poids entre le poids total de la créatine et/ou de l'allantoïne et le poids total en glycérine va de 1:6 à 1:2.

2. Composition selon la revendication 1 contenant en tant que combinaison de principes actifs, par rapport au poids total de la composition
a) 0,3 à 0,5 % en poids d'au moins un corps de cire choisi parmi la cire de candelilla, la cire de carnauba, la cire du Japon, la cire de sparte, la cire de liège, la cire d'huile de germes de riz, la cire de guarana, la cire d'Ouricuri, la cire de lignite, la cire de jojoba, la cire d'arganier, la cire de moringa oleifera, la cire d'olive, le beurre de karité, la cire de baies, la cire d'abeille, la cire de pépin de raison, la cire de noix de macadamia, la cire de tournesol, la cire de graine de coton, la cire de gomme-laque, le spermaceti, la lanoline, la graisse de Bürzel, la cérésine, l'ozokérite (cire naturelle), la vaseline, les cires de paraffine, les cires microcristallines, les cires d'ester de lignite, les cires de Sasol, les cires de jojoba hydrogénées et l'huile végétale hydrogénée ;
b) 0,35 à 0,5 % en poids d'au moins un polymère à base d'au moins un monomère d'acide acrylique et/ou d'au moins un ester d'un monomère d'acide acrylique ;
c) 0,2 à 0,3 % en poids d'au moins un alcool gras linéaire saturé non substitué comprenant 12 à 20 atomes de carbone ;
d) 7 à 10 % en poids d'au moins un émollient choisi parmi le PEG-30 glyceryl cocoate, le PEG-6 capryl/capric glycéride, le sorbitan sesquicaprylate, le sucrose cocoate, l'isomyl cocoate, le polyglyceryl-3 caprate, le PPG-3 myristyl éther, le PPG-11 stearyl éther, le cetearyl isononanoate, le cethyl éthylhexanoate, les caprylic/capric triglycérides, le decal cocoate, le diethylhexyl carbonate, le decyl oléate, le PPG-15 stearyl éther, l'octyldodécanol, l'isocetyl palmitate, l'isohexadécane, le cetearyl éthylhexanoate, l'isopropyl myristate, l'oleyl érucate, l'ethylhexyl palmitate, l'ethylhexyl stéarate, l'isopropyl palmitate, le PPG-14 butyl éther, la triisostéarine, le C12-15 alkylbenzoate, le cetyl ricénoleate, le glyceryl ricenoléate, le glyceryl stéarate, l'isocetyl palmitate, l'isocetyl stéarate, le tocopheryl linoléate, le methylheptyl isostéarate et des mélanges de ces derniers ;
e) 0,8 à 1,2 % en poids d'au moins un ester comprenant 20 à 34 atomes de carbone d'un acide gras linéaire, saturé non substitué et d'un alcool gras linéaire, saturé, non substitué ; et
f) 0,2 à 8 % en poids de glycérine et d'au moins un principe actif choisi parmi la créatine et/ou l'allantoïne, dans laquelle le rapport de poids entre le poids total de la créatine et/ou de l'allantoïne et le poids total en glycérine va de 1:6 à 1:2.

3. Composition selon l'une quelconque des revendications 1 ou 2 contenant en tant que principes actifs a) à f)
a) au moins un corps de cire animale et/ou végétale et au moins un corps de cire chimiquement modifié, dans laquelle le rapport de poids entre le poids total du ou des corps de cire animale et végétale utilisés et le poids total du ou des corps de cire chimiquement modifiés utilisés va de 1:2 à 2:1, de manière préférée est de 1:1 ;
b) au moins un polymère à base d'au moins un monomère d'acide acrylique et/ou d'au moins un ester d'un monomère d'acide acrylique, choisi parmi des homopolymères de l'acide acrylique et/ou des copolymères de l'acide acrylique et/ou l'ester d'alkyle de l'acide acrylique, de manière préférée l'ester d'alkyle en C10 à C30 de l'acide acrylique, et en particulier de manière préférée les acrylates/polymère croisé d'alkyl acrylate en C10-C30 ;
c) au moins un alcool gras linéaire, saturé non substitué comprenant 16 à 18 atomes de carbone, choisi parmi l'alcool stéaryle et l'alcool cétyle ;
d) au moins un émollient choisi parmi le PEG-30 glyceryl cocoate, le PEG-6 capryl/capric glycéride, le sorbitan sesquicaprylate, le sucrose cocoate, l'isoamyl cocoate, le polyglycéryl-3 caprate, le PPG-3 myristyl éther, le PPG-11 stearyl éther, le cetearyl isononanoate, le cetyl éthylhexanoate, les caprylic/capric triglycérides, le diethylhexyl carbonate, le decyl oléate, le PPG-15 stearyl éther, l'octyldodécanole, l'isocetyl palmitate, l'isohexadécane, le cetearyl éthylhexanoate, l'isopropyl myristate, l'oleyl érucate, l'ethylhexyl palmitate, l'ethylhexyl stéarate, l'isopropyl palmitate, le PPG-14 butyl éther, la triisostéarine, le C12-15 alkylbenzoate, le cetyl ricénoléate, le glyceryl ricénoléate, le glyceryl stéarate, l'isocetyl palmitate, l'isocetyl stéarate, le tocopheryl linoléate, le methylheptyl isostérate et des mélanges de ces derniers, en particulier de manière préférée l'isoamyl cocoate ;
e) au moins un ester comprenant 24 à 32, de manière préférée comprenant 28 atomes de carbone d'un acide gras linéaire, saturé, non substitué et d'un alcool gras linéaire, saturé, non substitué, dans laquelle le nombre des atomes de carbone dans le composant d'acide gras et le nombre des atomes de carbone dans le composant d'alcool gras ne diffèrent pas de plus de 2, de manière préférée de plus de 1, de manière particulièrement préférée d'aucun atome de carbone, en particulier choisi de manière préférée parmi le cetyl palmitate, le cetyl stéarate, le myristyl myristate et des mélanges de ces derniers ; et
f) de la glycérine et au moins un principe actif choisi parmi la créatine et/ou l'allantoïne, dans laquelle le rapport de poids entre le poids total de la créatine et/ou de l'allantoïne et le poids total de glycérine va de 1:6 à 1:2, dans laquelle la composition ne contient aucune autre substance, relevant des groupes de principes actifs mentionnés dans les groupes a) à f).

4. Composition selon l'une quelconque des revendications 1 à 3, **caractérisée en ce que** la composition contient en tant que composant f) de la créatine, de l'allantoïne et de la glycérine.

5. Composition selon l'une quelconque des revendications 1 à 4, dans laquelle la composition contient en tant que composant f) de la glycérine et au moins un autre principe actif choisi parmi la créatine et/ou l'allantoïne, **caractérisée en ce que** le rapport de poids entre le poids total de la créatine et de l'allantoïne et le poids total en glycérine va de 1:3,2 à 1:3,6.

6. Composition selon l'une quelconque des revendications 1 à 5, **caractérisée en ce que** le rapport de poids entre le poids total en composant de cire a) et le poids total en polymère du composant b) va de 2:1 à 1:2, est de manière préférée de 1:1.

7. Composition selon l'une quelconque des revendications 1 à 6, **caractérisée en ce que** la composition contient par ailleurs les principes actifs polyglycéryl-3-dicitrate/stéarate et urée.

8. Composition selon la revendication 7, **caractérisée en ce que** le polyglycéryl-3-dicitrate/stéarate et l'urée sont utilisés selon un rapport de poids allant de 100:1 à 150:1, de manière préférée de 125:1.

9. Composition selon l'une quelconque des revendications 1 à 8, contenant par ailleurs les principes actifs bétaïne et urée, **caractérisée en ce que** la bétaïne et l'urée sont utilisées selon un rapport de poids allant de 1:4 à 1:6.

10. Composition selon l'une quelconque des revendications 1 à 9, **caractérisée en ce que** la composition contient par ailleurs au moins 70 % en poids, de manière préférée au moins 80 % en poids d'eau.

11. Composition selon l'une quelconque des revendications 1 à 10, **caractérisée en ce que** la composition est sans composés de silicone et/ou sans parabène et/ou sans alkylparabène.

12. Procédé servant à fabriquer des compositions selon l'une quelconque des revendications 1 à 11, comprenant les étapes suivantes :
i) la fabrication d'une première phase en mélangeant les principes actifs a), c), une première partie de d) ainsi que e) ;
ii) la fabrication d'une deuxième phase en mélangeant au moins un des principes actifs mentionnés sous f) avec de l'eau ;
iii) la fabrication d'une troisième phase en mélangeant les principes actifs b) et la partie restante de d) ;
iv) l'homogénéisation de la première et de la deuxième phase par une agitation ;
v) le mélange de la composition obtenue à l'étape iv) avec la troisième phase ;
vi) éventuellement l'ajout de parfum, d'un agent de conservation, de betaïne, d'urée et/ou d'autres substances à la composition obtenue à l'étape v) ; et
vii) le réglage de la valeur pH ; et
viii) éventuellement la mise sous vide de la composition à température ambiante.

13. Utilisation de compositions selon l'une quelconque des revendications 1 à 11 en tant que crème pour la peau, en particulier en tant que crème pour les mains et les avant-bras.

14. Utilisation cosmétique de compositions selon l'une quelconque des revendications 1 à 11 en tant que soin hydratant, en particulier en tant que soin hydratent de longue durée, pour la peau.

15. Utilisation cosmétique de compositions selon l'une quelconque des revendications 1 à 11 servant à protéger les peaux abîmées.
